# EUROPEAN PATENT SPECIFICATION

(11) **EP 3 229 780 B1**
(45) Date of publication and mention of the grant of the patent: **28.11.2018**
(21) Application number: 15823780.0
(22) Date of filing: 11.12.2015
(51) Int. Cl.: A61K 9/00, A61K 47/36, A61K 31/573

(54) **OPHTHALMIC COMPOSITIONS FOR USE IN THE TREATMENT OF THE DRY EYE SYNDROME**
OPHTHALMISCHE ZUSAMMENSETZUNGEN ZUR VERWENDUNG BEI DER BEHANDLUNG DES TROCKENEN AUGES
COMPOSITIONS OPHTALMIQUES DESTINÉES À ÊTRE UTILISÉES DANS LE TRAITEMENT DU SYNDROME DE L'OEIL SEC

(30) Priority: 12.12.2014 IT MI20142126
(43) Date of publication of application: 18.10.2017
(73) Proprietor: Alfa Intes Industria Terapeutica Splendore S.r.l., 80026 Casoria (NA) (IT)
(72) Inventor: GELSOMINO, Lucia, 80026 Casoria (NA) (IT)
(74) Representative: Porta, Checcacci & Associati S.p.A
(86) International application number: PCT/IB2015/059533
(87) International publication number: WO 2016/092513

(56) References cited:
- DE-B1- 1 617 628
- GB-A- 1 386 864
- US-A- 5 236 906
- US-A1- 2005 196 370

## Description

### Field of the invention

The present invention relates to new ophthalmic pharmaceutical compositions for use in the treatment of the dry eye syndrome.

### Background of the invention

Dry eye syndrome is among the most frequent pathological conditions in ophthalmology. Up to 20% of adults over 40 years old have dry eye symptoms.

Such syndrome is an eye disease that consists of an insufficient production, both from a quantitative and qualitative standpoint, of the lacrimal film - which mainly has a moistening function of the eye surface - by the related glands. Such pathology can be damaging since it can cause lesions to the external structures of the eye. The lacrimal film, in fact, tends to break up, with consequent exposure to dehydration of the anterior corneal epithelium and of the palpebral conjunctiva.

The patient affected by dry eye syndrome has disorders such as conjunctival reddening, burning, sensation of foreign body in the eye, photophobia, difficulty in opening the eyelid upon wakening, and in the most serious cases, pain and visual dimming.

Such eye disease requires a treatment of the patient with lacrimal substitutes (artificial tears or moistening gels) having mucomimetic and pseudoplastic properties.

### Related art

Often, the chronic condition of insufficient lacrimal production exposes the corneal surface to attack by external agents present in the environment capable to trigger annoying inflammatory processes on the conjunctiva and on the corneal epithelium. In such conditions, the use of only lacrimal substitutes is no longer sufficient to restore physiological conditions. The ophthalmologist, therefore, is forced to also prescribe a therapy based on corticosteroids, which perform an important role in controlling inflammation.

However, these corticosteroid-based therapies may be limited over time due to the known side effects that corticosteroids cause in the eye. Once the corticosteroids have passed beyond the corneal barrier and penetrated inside the eye, in fact, they can cause the onset of cataract and an increase of the intraocular pressure. These are very harmful effects that therefore limit the use of these drugs to a very limited, hence non-resolutive, time period. The use of eye drops based on hydrocortisone sodium phosphate at 0.33%, commercially available with the name of Cortivis® (Medivis S.r.1.), is known.

In the patient information leaflet of such a drug, it is reported that the treatment with corticosteroids must not be repeated or prolonged without suitable medical controls, in order to prevent the onset of intraocular pressure and unexpected infections or the formation of cataract.

Among the most frequent side effects in case of repeated or prolonged use of a corticosteroid, in fact, the following are mentioned: rise of the intraocular pressure with possible development of glaucoma, formation of posterior subcapsular cataract, thickening of the sclera, mydriasis, relaxation of the eyelids.

Since a systemic absorption as a consequence of topical use of corticosteroids can be verified, adverse systemic effects can also occur such as cephalea, hypotension, rhinitis and pharyngitis.

Recently, there has been a certain trend to use corticosteroids in combination with cyclosporin. The rational for this combined treatment lies in the fact that, contrary to the corticosteroid that exerts a quick and intense control on the inflammation of the eye surface, cyclosporin requires more time for it to reach its maximum effect (from several weeks to months).

International patent application WO 2006/073786 discloses ophthalmic pharmaceutical compositions comprising a corticosteroid and a cyclosporin. Preferably, the corticosteroid is loteprednol etabonate.

International patent application WO 03/011249 discloses an ophthalmic preparation containing N-acetylcysteine having a mucolytic action, adapted for the administration in eye drops, wherein the N-acetylcysteine is neutralized with base DEAE-dextrane in order to reach pH levels preferably between 6.0 and 7.5, and having a physiologically acceptable osmolarity less than 320 mOsm/kg.

GB patent No. 1 386 864 discloses ophthalmic pharmaceutical compositions for the topical application to the eye having an enhanced drug action and/or prolonged action duration, comprising a pharmacologically active ophthalmic agent and from 0.1 to 5.0% by weight of microcrystalline collagen based on the total weight of the composition. The ophthalmic agent may be chloramphenicol, carbomycin, polymyxin, erythromycin, dihydrostreptomycin, neomycin, aureomycin, terramycin, bacitracin, penicillin, ampicillin, atropine, homatropine, scopolamine, cyclopentalate, tropicamide, oxyphenonium, tetracaine, proparacaine, benoxinate, cocaine, procaine, lidocaine, epinephrine, isoniazid, nitrofurans, sulphonamides such as sulphanilamide, sulphapyridine, sulphadiazine, sulphathiazole, sulphaceamide, phenylephrine, ephedrine, gentian violet, acriflavine, silver nitrate, acetylcholine, carbachol, pilocarpine, esedrine, echothiophate iodide, demercarium, dihydroergocornine, tolazoline, zinc sulphate, chlorobutanol, phenyl butazone, tetraethylammonium chloride, hexamethonium, noradrenaline, physostigmine, cortisone, hydrocortisone, prednisolone, dexamethasone, triamicinolone, methyl prednisolone, argyrol, phenylmercuric nitrate, chlorazene, mercurochrome, iodine or a quaternary ammonium germicide, or mixtures thereof.

GB patent No. 1 149 648 describes a process for sterilizing steroids, like dexamethasone or its phosphate, acetate or tert-butyl-acetate, hydrocortisone, methyl prednisolone acetate, betamethasone acetate, and triamcinolone acetonide, as well as sterile compositions for parenteral use or ophthalmic application which contain in addition to one or more of the aforesaid ingredients, one or more suspending agents, per example sodium carboxymethylcellulose, hydroxyethyl cellulose or sorbitol; chelating agents, e.g. N-ethylene-diamine-tetra-acetate; preservatives, e.g. Na-bisulphite or benzalkonium chloride; lecithin; creatinine; alcohols; benzyl alcohol; and lidocaine.

US patent application No. 2005/0196370 discloses stable oil-in-water emulsions which contain an emollient for the treatment of dry eye constituted by a water-soluble polymer such as sodium hyaluronate.

In one embodiment, such compositions can include a second therapeutic agent in addition to the water-soluble polymer. Example of such second therapeutic agent are anti-infective and anti-microbial substances, anti-allergy agents, antihistaminics, anti-hypertensive agents and decongestants, anti-inflammatories, such as cortisone, hydrocortisone, hydrocortisone acetate, betamethansone, dexamethasone, dexamethasone sodium phosphate, prednisone, methylprednisolone, medrysone, fluorometholone, fluocortolone, prednisolone, prednisolone sodium phosphate, triamcinolone, sulindac, salts and corresponding sulfides thereof, and the like and mixtures thereof, non-steroid anti-inflammatory drugs (NSAID), miotics and anticholinergics, mydriatics, antiglaucoma drugs, antiparasitic compounds and/or anti-protozoal compounds, antiviral compounds, carbonic anhydrase inhibitors, anti-fungal agents, pain-relieving and anesthetic agents, ophthalmic diagnostic agents, ophthalmic agents used as adjuncts in surgery, chelating agents, immunosuppressive agents and anti-metabolites, angiostatic agents, muco-secretogogue agents, proteins and growth factors, vitamins and vitamin derivatives, and combinations of such therapeutic agents such as antibiotic/anti-inflammatory (neomycin sulfate-dexamethasone sodium phosphate), quinolones-NSAIDs and the like.

### Summary of the invention

The Applicant has therefore addressed the problem of providing ophthalmic compositions for the treatment of the dry eye syndrome containing a corticosteroid, which possess a suitable anti-inflammatory activity on the eye surface without however triggering, in particular in the case of a prolonged treatment time, the side effects that corticosteroids can cause in the eye (for example, the onset of cataract and the rise of intraocular pressure). The Applicant has surprisingly and experimentally found that it is possible to achieve both the desired anti-inflammatory effect on the eye surface, and to prevent an absorption of the corticosteroid - even in the case of a prolonged treatment time - by formulating an ophthalmic pharmaceutical composition for use in the treatment of the dry eye syndrome which comprises, in combination:
i) hydrocortisone or a pharmaceutically acceptable salt thereof in an amount equal to or greater than 0.0005% by weight and lower than 0.003% by weight, and
ii) a lacrimal substitute.

In the following description and in the subsequent claims, the term lacrimal substitute, is used to indicate a compound or an association of compounds having viscosizing properties and capable to impart pseudoplastic characteristics to the composition. Within the context of the ophthalmic pharmaceutical composition according to the invention, the lacrimal substitute carries out the functions of improving the stability of the lacrimal film, protecting the eye surface from the attack of external agents and of imparting at the same time to the composition viscosity characteristics capable of "mechanically" retaining the corticosteroid in contact with the cornea.

The Applicant has unexpectedly found that with an ophthalmic pharmaceutical composition formulated as described above, no penetration of the corticosteroid into the anterior chamber of the eye occurs, thereby substantially eliminating the risk of an onset of the above-mentioned side effects, such as glaucoma and cataract, related to the association of a lacrimal substitute with a corticosteroid.

All this is clearly in contrasts with what would be expected based on the literature data, which widely show that the use of lacrimal substitutes in ophthalmic liquid formulations is capable of increasing the ocular bioavailability of the carried drugs. Notwithstanding what is reported in the literature, the Applicant has instead unexpectedly found that the use of hydrocortisone in the ophthalmic composition, in the above-indicated specific and limited amounts, is capable both of preventing an absorption of the hydrocortisone by the eye, and of simultaneously ensuring an effective anti-inflammatory topical action in the treatment of dry eye.

In an entirely unexpected manner, these technical effects are achieved notwithstanding the simultaneous presence in the ophthalmic formulation of a lacrimal substitute - which increases due to its own nature the ocular bioavailability of the corticosteroid employed - even in the case of prolonged treatment times, such as those necessary for alleviating the symptoms of the dry eye syndrome of chronic type.

Advantageously, therefore, the aforementioned specific and very limited concentration range of hydrocortisone is effective for combating the inflammatory process on the eye surface, without penetration phenomena inside the eye structure, thus ensuring user safety.

### Detailed description of the currently preferred embodiments of the invention

Within the framework of the present description and in the subsequent claims, except where otherwise indicated, all the numerical entities expressing amounts, parameters, percentages, and so forth, are to be understood as being preceded in all instances by the term "about". Also, all ranges of numerical entities include all the possible combinations of the maximum and minimum values and include all the possible intermediate ranges, in addition to those specifically indicated hereinbelow.

In a preferred embodiment, the ophthalmic pharmaceutical composition of the invention is for topical use in the treatment of the dry eye syndrome.

In a preferred embodiment, the pharmaceutically acceptable salt of hydrocortisone is selected from the group comprising sodium phosphate, acetate, sodium succinate, butyrate and valerate. Sodium phosphate is the most preferred pharmaceutically acceptable salt.

In a particularly preferred embodiment, the hydrocortisone or its pharmaceutically acceptable salt is present in an amount equal to or greater than 0.0008 and equal to or less than 0.002, more preferably equal to 0.001% by weight.

Preferably, the lacrimal substitute also has characteristics capable to render the ophthalmic pharmaceutical composition mucoadhesive, meaning with this term the ability to adhere to the mucin naturally present in the lacrimal film.

Advantageously, the lacrimal substitute allows to achieve in such a case also a strengthening of the mucous layer of the lacrimal film and contributes to restore the function of such a film.

Preferably, the lacrimal substitute also has characteristics capable to render the ophthalmic pharmaceutical composition mucomimetic, meaning with this term the capacity to have a behavior similar to that of mucin.

Advantageously, the lacrimal substitute also allows in such a case to have an ophthalmic pharmaceutical composition capable of substituting mucin deficiencies in the so-called "dry spots" and of retaining water so as to ensure a correct hydration of the eye surface. In a particularly preferred embodiment, the lacrimal substitute has characteristics capable to render the ophthalmic pharmaceutical composition pseudoplastic, mucoadhesive and mucomimetic, so to "mechanically" retain the corticosteroid in contact with the cornea. Preferably, the lacrimal substitute is selected from the group comprising: glycosaminoglycans, cellulose derivatives, polyacrylic acid derivatives, water-soluble polymers that are physiologically compatible with the eye, and mixtures thereof. Preferably, the glycosaminoglycans which can be used in the present invention are selected from the group comprising: sodium hyaluronate (SH), hyaluronic acid, chondroitin sulfate, and mixtures thereof.

In a particularly preferred embodiment, the glycosaminoglycan is sodium hyaluronate (SH).

Advantageously, the SH - which is naturally present in the eye - renders the ophthalmic composition pseudoplastic, mucoadhesive and mucomimetic.

Preferably, the cellulose derivatives which can be used in the present invention are selected from the group comprising: carboxymethylcellulose (CMC), hydroxypropylmethylcellulose (HPMC), methylcellulose (MC), hydroxyethylcellulose (HEC) and hydroxypropylcellulose (HPC) and mixtures thereof.

Preferably, the derivatives of polyacrylic acid which can be used in the present invention are selected from: carbopol, polycarbophil and the like, and mixtures thereof. Preferably, the water-soluble polymers which can be used in the present invention are selected from: polyvinyl alcohol (PVA), dextran, polyvinylpyrrolidone (PVP), polysorbate, and mixtures thereof.

In a particularly preferred embodiment, the lacrimal substitute is present in the composition according to the invention in an amount comprised between 0.1 and 0.5% by weight, preferably 0.2% by weight.

Preferably, the lacrimal substitute of the ophthalmic pharmaceutical composition according to the invention has a molecular weight comprised between 500 and 1200 kDa. Advantageously, such medium-low molecular weight is more tolerable for the patient, in particular for use in the treatment of the dry eye syndrome of chronic type.

In a further preferred embodiment, the ophthalmic pharmaceutical composition according to the present invention has a pH comprised between 5.5 and 7.5.

Preferably, the ophthalmic pharmaceutical composition according to the invention has a physiologically acceptable osmolarity, preferably comprised between 240 and 330 mOsm/kg.

The ophthalmic compositions for use in the treatment of the dry eye syndrome according to the invention can contain one or more of the other possible ingredients known in the pharmaceutical art for ophthalmic preparations.

In particular, the ophthalmic compositions can further contain at least one component selected from the group comprising buffering agents, osmolarity regulators, and mixtures thereof.

The buffering agents or acidity regulators help to maintain the pH of the ophthalmic products as close as possible to the physiological pH. This action is essential for achieving a good tolerability of the ophthalmic preparations and for preserving the effectiveness thereof.

Examples of buffering agents which can be used in the present invention are selected from the group comprising acetic acid, sodium phosphate, sodium hydroxide, sodium citrate, sodium carbonate, sodium borate, sodium bicarbonate, potassium phosphate, potassium citrate, potassium carbonate, boric acid, hydrochloric acid, acetic acid and magnesium chloride, and mixtures thereof.

Examples of substances which regulate the osmolarity, rendering the ophthalmic compositions isotonic with the tears, are selected from the group comprising potassium chloride, propylene glycol, sodium chloride, glycerin, dextrose, dextran 40 and 70, and mixtures thereof.

As is customary in the art, the ophthalmic compositions according to the invention can contain other adjuvants, including preserving agents, sequestering agents, antimicrobial agents, and mixtures thereof.

These adjuvants can be absent in the ophthalmic compositions formulated in the form of eye drops, if single-dose packages without preservatives are used.

In a preferred embodiment, the ophthalmic compositions for use in the treatment of the dry eye syndrome according to the invention are conveniently prepared in the form of an aqueous solution or suspension in a pharmaceutically acceptable ophthalmic carrier. Preferably, the compositions according to the invention are prepared in the form of eye drops, gel or cream.

According to a preferred embodiment thereof, the present invention relates to an ophthalmic pharmaceutical composition as described above, for use in the treatment of the dry eye syndrome of the chronic type.

As mentioned above, in fact, with the ophthalmic pharmaceutical composition of the invention, no penetration of the corticosteroid into the anterior chamber of the eye occurs, with a substantial elimination of the risk of an onset of the above-mentioned side effects, such as glaucoma and cataract, even in the case of a prolonged topical treatment of the eye.

In order to verify the performances of the ophthalmic compositions according to the invention, various experiments were carried out some results of which are reported hereinbelow.

### EXAMPLES

### EXAMPLE 1

An ophthalmic pharmaceutical composition in the form of eye drops according to the invention was prepared by mixing hydrocortisone sodium phosphate and sodium hyaluronate with other conventional ingredients according to a method known to a person skilled in the art and thereafter was subjected to sterilization by means of filtration. In this way, an eye drop formulation was obtained having the composition reported in the following Table 1.

**TABLE 1**

| **Components** | **Quantity [mg]** |
|---|---|
| Hydrocortisone sodium phosphate | 1 |
| Sodium hyaluronate (SH) | 200 |
| Sodium chloride | 745 |
| Potassium chloride | 180 |
| Trisodium citrate dihydrate | 38 |
| Magnesium chloride hexahydrate | 13 |
| Na₂HPO₄ dodecahydrate | 53 |
| Na₂HPO₄ monohydrate | 8 |
| Purified H₂O | balance to 100 ml |

### Pharmacokinetic study to evaluate the safety of the ophthalmic compositions according to the invention

Four different eye drop formulations containing - the other ingredients being the same - different concentrations of hydrocortisone:
**Group 1** - Formulation A: 0.001% by weight of hydrocortisone sodium phosphate (Example 1 in accordance with the present invention);
**Group 2** - Formulation B: 0.003% by weight of hydrocortisone sodium phosphate (reference sample);
**Group 3** - Formulation C: 0.005% by weight of hydrocortisone sodium phosphate (reference sample);
**Group 4** - Formulation D: 0.33% by weight of hydrocortisone sodium phosphate (reference sample);
   were administered in each eye of male New Zealand White rabbits (8 rabbits/group), by means of instillation in the conjunctival fornix of one drop of eye drops formulation, 4 times per day, in order to evaluate the absorption thereof in the aqueous humor.

The reference formulations B, C and D contain the same ingredients as formulation A (Example 1), except for the amount of active ingredient.

Samples of aqueous humor (150 µl) were collected, by means of a suitable apparatus, from both eyes for each of the 32 male rabbits at 15, 30, 60 and 90 minutes after the fourth dose on the first day of treatment. Before collecting the samples, the animals were sacrificed with an intravenous injection of 20 mg/kg sodium thiopental followed by an administration of Tanax® (Intervet, Milan).

The samples of aqueous humor were analyzed according to the validation protocol UNIF-2012-01.

100 µl of aqueous humor samples were aliquoted into a 1.5 ml polypropylene microcentrifuge tube, followed by addition of 5 µl of 2 µg/ml freshly prepared internal standard solution. After vigorous mixing for 30 seconds, the samples were aliquoted by the addition of 1000 µl of ethyl acetate followed by vigorous mixing for 2 min and centrifugation at 12,000 rpm at +4°C for 25 min, aliquots of 850 µl of organic layer were transferred into a single glass tube and dried under vacuum at 50°C using a Büchi vacuum system for 60 min.

The residue was reconstituted with 100 µl of mobile phase (water, with 0.1% HCOOH/acetonitrile with 0.1% HCOOH, 75/25, v/v). The tubes were capped, vigorously mixed and centrifuged at 4100 rev/min at + 4°C for 10 min.

The final extracts were transferred into an autosampler vial and 4 µl were injected into the LC-MS / MS system.

The obtained results are reported in the following Table 2.

**Table 2**

| | **Hydrocortisone concentration in the aqueous humor (ng/ml)** | | | |
|---|---|---|---|---|
| | **15'** | **30'** | **60'** | **90'** |
| **Group 1** | 0.00 | 0.00 | 0.00 | 0.00 |
| | 0.00 | 0.00 | 0.00 | 0.00 |
| | 0.00 | 0.00 | 0.00 | 0.00 |
| | 0.00 | 0.00 | 0.00 | 0.00 |
| **Group 2** | 0.00 | 7.72 | 8.81 | 5.75 |
| | 3.60 | 8.79 | 7.48 | 4.55 |
| | 5.09 | 12.63 | 4.23 | 4.22 |
| | 4.28 | 13.26 | 4.99 | 4.89 |
| **Group 3** | 8.12 | 18.29 | 23.15 | 21.46 |
| | 8.75 | 17.41 | 24.81 | 19.85 |
| | 14.46 | 18.85 | 24.43 | 19.22 |
| | 12.96 | 20.54 | 23.79 | 19.30 |
| **Group 4** | 58.38 | 68.61 | 54.70 | 56.19 |
| | 60.10 | 69.01 | 56.80 | 56.73 |
| | 59.77 | 68.38 | 55.06 | 56.16 |
| | 60.70 | 70.60 | 55.52 | 55.00 |

As shown in Table 2, no hydrocortisone level was found in the aqueous humor after topical administration of the composition, comprising a hydrocortisone concentration of 0.001% by weight, according to the present invention, advantageously demonstrating that the active ingredient does not pass beyond the corneal barrier. On the contrary, the results obtained with the comparative compositions comprising concentrations of hydrocortisone equal to or greater than the maximum limit of 0.003% by weight, respectively of 0.003%, 0.005% and 0.33% by weight, have shown that the drug passes beyond the corneal barrier and that the bioavailability in the aqueous humor is dose-dependent.

### Efficacy studies in experimental models of dry eye syndrome in rabbits

### Study I (Concanavalin A - induced model)

Six male New Zealand Harlan rabbits were randomly distributed in 2 groups (3 animals/group) according to the following scheme:
**Group I -** Control formulation: carrier containing sodium hyaluronate (SH) at 0.2% by weight;
**Group II** - Formulation A: ophthalmic pharmaceutical composition in the form of eye drops containing 0.001% by weight of hydrocortisone sodium phosphate (Example 1 according to the present invention).

The rabbits were anesthetized by means of intravenous injection of tiletamine HCl and zolazepam HCl (Zoletil®; Virbac). Each rabbit received an injection of concanavalin A (ConA) in the lacrimal gland. The injections were carried out by means of slight retraction of the lower eyelid and by inserting the needle at about 1 cm from the corner of the eye in the suborbital space to a depth of about 6 mm. A single 30 µl volume of saline solution or ConA was injected in the lacrimal gland by using a 30-gauge needle and Hamilton syringe. The rabbits were then sacrificed with an intravenous injection of Tanax® (Intervet, Milan). The injection of 300 µg of ConA in the rabbit lacrimal glands produces lacrimal irregularities and, on the eye surface, characteristics typical of the dry eye syndrome.

Each rabbit was administered the drops (30 µl) of control formulation or of formulation A, 4 times per day, for three days.

On the third day after the injection of ConA, the levels of TNF-α, MMP-9 and IL-8 in the lacrimal fluid were measured by means of an ELISA assay.

It is well known, in fact, that after the induction with ConA, the levels of MMP-9 and cytokines dramatically increase in the lacrimal liquid, in a manner similar to what happens in patients with dry eye (Luo et al. IOVS 2004, 45, 4293-4301).

The data are shown in Table 3. The topical treatment with an ophthalmic pharmaceutical composition containing hydrocortisone at 0.001% by weight, according to the present invention, significantly reduced (p <0.05; p <0.001) the levels of TNF-α and IL-8 with respect to the carrier (group treated with 0.2% SH). Even if not statistically significant, a reduction of the levels of MMP-9 with respect to the carrier was also observed.

**Table 3**

| **Biomarkers (lacrimal levels) pg/ml** | **Carrier (average±DS)** | **Hydrocortisone (average±DS)** |
|---|---|---|
| TNF-alpha | 289.2±28.6 | 281.2±12.3* |
| IL-8 | 627±57 | 221±30** |
| MMP-9 | 11±1.8 | 9.3±2.8 |

| | | |
|---|---|---|
| *p<0.05; **p<0.001; the statistical analysis was carried out by means of the t test. | | |

In addition, on the first, second and third day after injection of ConA, the lacrimal volume was evaluated by means of the Schirmer test and the lacrimal film breakup (TBUT). Schirmer strips were carefully placed in the posterior inferior (i.e. temporal) fornix for 60 seconds, and the wet zone was read in millimeters as lacrimal volume index. The lacrimal film breakup TBUT (Tear breakup time) was determined after instillation of 5 µl of 2% sodium fluorescein in sterile PBS on the lower eyelid. The fluorescein was distributed on the cornea by means of a manual winking of the eyelids. The eye opening time was recorded under a slit lamp (Sbisà, Florence, Italy) with cobalt blue filter for improving the fluorescein signals, up to the appearance of the first black point or strip on the cornea (dry zones which lose fluorescence).

The data are shown in Table 4. The topical treatment with an ophthalmic pharmaceutical composition containing hydrocortisone at 0.001% by weight, according to the present invention, is capable of significantly maintaining (p<0.05; p<0.01) the lacrimal volume and the lacrimal integrity measured by means of the Schirmer test and of the TBUT, respectively.

**Table 4**

| **Lacrimal volume (mm)** | **Carrier (average±DS)** | **Hydrocortisone (average±DS)** |
|---|---|---|
| Day 1 | 4.5±1 | 7.1±2.1* |
| Day 2 | 4.1±0.7 | 7.5±1.5* |
| Day 3 | 4.5±1.2 | 7.8±1.2* |

| **TBUT (seconds)** | **Carrier (average±DS)** | **Hydrocortisone (average±DS)** |
|---|---|---|
| Day 1 | 10±1 | 22±1.7** |
| Day 2 | 9±0.9 | 20.8±2** |
| Day 3 | 8±0.9 | 20±1** |

| | | |
|---|---|---|
| *p<0.05; **p<0.01; the statistical analysis was carried out by means of the t test. | | |

### Study II (Atropine-induced model)

Six male New Zealand Harlan rabbits were randomly distributed in 2 groups (3 animals/group) according to the following scheme:
**Group I -** Control formulation: carrier containing sodium hyaluronate (SH) at 0.2% by weight;
**Group II -** Formulation A: ophthalmic pharmaceutical composition in the form of eye drops containing 0.001% by weight of hydrocortisone sodium phosphate (Example 1 according to the present invention).

An ophthalmic solution of 1% atropine sulphate (30 µl) was instilled in the inferior conjunctival sac of each eye, four times over 12 hours, in order to cause an experimental dry eye condition (Burgalassi et al., Ophthalmic Res. 31, 229-235, 1999). Fifteen minutes after the administration of each dose of atropine sulphate, each eye received 30 µl of hydrocortisone or carrier.

24 hours after the first instillation of atropine, the lacrimal volume was evaluated by means of the Schirmer test. Schirmer strips were carefully placed in the posterior inferior (i.e. temporal) fornix for 60 seconds, and the wet zone was read in millimeters as lacrimal volume index. 24 hours after the first instillation of atropine, the lacrimal film breakup TBUT (Tear breakup time) was also determined after instillation of 5 µl of 2% sodium fluorescein in sterile PBS on the lower eyelid. The fluorescein was distributed on the cornea by means of a manual winking of the eyelids. The eye opening time was recorded under a slit lamp (Sbisà, Florence, Italy) with cobalt blue filter for improving the fluorescein signals, up to the appearance of the first black point or strip on the cornea (dry zones which lose fluorescence).

The data are shown in Table 5. The topical treatment with an ophthalmic pharmaceutical composition containing hydrocortisone at 0.001% by weight, according to the present invention, is capable of significantly maintaining (p<0.01) the lacrimal volume and the lacrimal integrity measured by means of the Schirmer test and of the TBUT, respectively.

**Table 5**

| **Parameter** | **Carrier (average±DS)** | **Hydrocortisone (average±DS)** |
|---|---|---|
| Lacrimal volume (mm) | 7±1.4 | 10.3±0.8* |
| TBUT (seconds) | 7.1±1.7 | 19.3±2.6* |

| | | |
|---|---|---|
| *p<0.01; the statistical analysis was executed by means of the t test. | | |

A concentration of 0.001% by weight of hydrocortisone in an ophthalmic pharmaceutical composition according to the present invention therefore proved effective in opposing the inflammatory processes induced on the animal, since the simultaneous presence of sodium hyaluronate, preferably in the concentration of 0.2% by weight, imparts pseudoplastic, mucoadhesive and mucomimetic characteristics to the ophthalmic composition which render the latter capable of "mechanically" retaining the hydrocortisone in contact with the cornea, increasing the residence time thereof on the eye surface and hence also increasing the bioavailability.

In conclusion, the obtained data demonstrate that the treatment of the dry eye syndrome with an ophthalmic pharmaceutical composition containing hydrocortisone at 0.001% by weight in combination with a suitable amount of a suitable lacrimal substitute (for example, and preferably, sodium hyaluronate) is effective both in the ConA-induced and in the atropine-induced dry eye animal model.

In particular, in Study I, the treatment with an ophthalmic pharmaceutical composition containing hydrocortisone at 0.001% by weight in combination with a suitable amount of a suitable lacrimal substitute (for example, and preferably, sodium hyaluronate) has proven capable of reducing the levels of MMP-9, TNF-α and IL-8 in the tears and of maintaining a physiological level of lacrimal volume, as well as lacrimal integrity.

The topical treatment with an ophthalmic pharmaceutical composition containing hydrocortisone at 0.001% by weight in combination with a suitable amount of a suitable lacrimal substitute (for example, and preferably, sodium hyaluronate) was also capable of maintaining the lacrimal function at physiological levels, even in the atropine-induced dry eye animal model.

## Claims

1. An ophthalmic pharmaceutical composition for use in the treatment of the dry eye syndrome comprising hydrocortisone or a pharmaceutically acceptable salt thereof in an amount equal to or greater than 0.0005% by weight and less than 0.003% by weight and a lacrimal substitute.

2. An ophthalmic pharmaceutical composition for use in the treatment of the dry eye syndrome according to claim 1, wherein the pharmaceutically acceptable salt of hydrocortisone is selected from the group comprising sodium phosphate, acetate, sodium succinate, butyrate and valerate.

3. An ophthalmic pharmaceutical composition for use in the treatment of the dry eye syndrome according to claim 1 or 2, wherein the hydrocortisone or the pharmaceutically acceptable salt thereof is present in an amount equal to or greater than 0.0008 and equal to or less than 0.002, preferably equal to 0.001% by weight.

4. An ophthalmic pharmaceutical composition for use in the treatment of the dry eye syndrome according to any one of claims 1 to 3, wherein the lacrimal substitute is selected from the group comprising glycosaminoglycans, cellulose derivatives, derivatives of polyacrylic acid, water-soluble polymers physiologically compatible with the eye, and mixtures thereof.

5. An ophthalmic pharmaceutical composition for use in the treatment of the dry eye syndrome according to claim 4, wherein the glycosaminoglycan is sodium hyaluronate.

6. An ophthalmic pharmaceutical composition for use in the treatment of the dry eye syndrome according to any one of claims 1 to 5, wherein the lacrimal substitute is present in an amount comprised between 0.1 and 0.5% by weight, preferably 0.2% by weight.

7. An ophthalmic pharmaceutical composition for use in the treatment of the dry eye syndrome according to any one of claims 1 to 6, wherein the lacrimal substitute has a molecular weight comprised between 500 and 1200 kDa.

8. An ophthalmic pharmaceutical composition for use in the treatment of the dry eye syndrome according to any one of claims 1 to 7, having a pH comprised between 5.5 and 7.5.

9. An ophthalmic pharmaceutical composition for use in the treatment of the dry eye syndrome according to any one of claims 1 to 8, having a physiologically acceptable osmolarity, preferably comprised between 240 and 330 mOsm/kg.

10. An ophthalmic pharmaceutical composition for use in the treatment of the dry eye syndrome according to any one of claims 1 to 9, further comprising at least one component selected from the group comprising buffering agents, osmolarity regulators, and mixtures thereof.

11. An ophthalmic pharmaceutical composition for use in the treatment of the dry eye syndrome according to any one of claims 1 to 10, further comprising preserving agents, antimicrobial agents, sequestering agents, and mixtures thereof

12. An ophthalmic pharmaceutical composition for use in the treatment of the dry eye syndrome according to any one of claims 1 to 11, in the form of an aqueous solution or suspension in a pharmaceutically acceptable ophthalmic carrier.

13. An ophthalmic pharmaceutical composition for use in the treatment of the dry eye syndrome according to claim 12, in the form of eye drops, gel or cream.

14. An ophthalmic pharmaceutical composition for use in the treatment of the dry eye syndrome according to any one of claims 1 to 13, wherein the dry eye syndrome is of the chronic type.

## Patentansprüche

1. Ophtalmische pharmazeutische Zusammensetzung zur Verwendung bei der Behandlung des Syndroms des trockenen Auges, umfassend Hydrocortison oder ein pharmazeutisch annehmbares Salz davon in einem Anteil von größer oder gleich 0,0005 Gew.-% oder weniger als 0,003 Gew.-% sowie einen Tränenersatz.

2. Ophtalmische pharmazeutische Zusammensetzung zur Verwendung bei der Behandlung des Syndroms des trockenen Auges nach Anspruch 1, wobei das pharmazeutisch annehmbare Hydrocortisonsalz ausgewählt ist aus der Gruppe umfassend Natriumphosphat, Acetat, Natriumsuccinat, Butyrat und Valerat.

3. Ophtalmische pharmazeutische Zusammensetzung zur Verwendung bei der Behandlung des Syndroms des trockenen Auges nach Anspruch 1 oder 2, wobei das Hydrocortison oder das pharmazeutisch annehmbare Salz davon in einem Anteil von größer oder gleich 0,0008 Gew.-% oder weniger als 0,002 Gew.-%, vorzugsweise gleich 0,001 Gew.-% vorliegt.

4. Ophtalmische pharmazeutische Zusammensetzung zur Verwendung bei der Behandlung des Syndroms des trockenen Auges nach einem der Ansprüche 1 bis 3, wobei der Tränenersatz ausgewählt ist aus der Gruppe umfassend Glycosaminoglycane, Cellulosederivate, Polyacrylsäurederivate, physiologisch mit dem Auge verträgliche, wasserlösliche Polymere und Gemische davon.

5. Ophtalmische pharmazeutische Zusammensetzung zur Verwendung bei der Behandlung des trockenen Auges nach Anspruch 4, wobei das Glycosaminglycan ein Natriumhyaluronat ist.

6. Ophtalmische pharmazeutische Zusammensetzung zur Verwendung bei der Behandlung des Syndroms des trockenen Auges nach einem der Ansprüche 1 bis 5, wobei der Tränenersatz in einem Anteil von 0,1 bis 0,5 Gew.-%, vorzugsweise von 0,2 Gew.-% vorliegt.

7. Ophtalmische pharmazeutische Zusammensetzung zur Verwendung bei der Behandlung des Syndroms des trockenen Auges nach einem der Ansprüche 1 bis 6, wobei der Tränenersatz ein Molekulargewicht im Bereich von 500 bis 1200 kDa aufweist.

8. Ophtalmische pharmazeutische Zusammensetzung zur Verwendung bei der Behandlung des Syndroms des trockenen Auges nach einem der Ansprüche 1 bis 7, die einen pH-Wert im Bereich 5,5 bis 7,5 aufweist.

9. Ophtalmische pharmazeutische Zusammensetzung zur Verwendung bei der Behandlung des Syndroms des trockenen Auges nach einem der Ansprüche 1 bis 8, die eine physiologisch annehmbare Osmolarität, vorzugsweise im Bereich von 240 bis 330 mOsm/kg aufweist.

10. Ophtalmische pharmazeutische Zusammensetzung zur Verwendung bei der Behandlung des Syndroms des trockenen Auges nach einem der Ansprüche 1 bis 9, weiter umfassend wenigstens einen Bestandteil ausgewählt aus der Gruppe umfassend Puffersubstanzen, Osmolaritätsregulatoren und Gemische davon.

11. Ophtalmische pharmazeutische Zusammensetzung zur Verwendung bei der Behandlung des Syndroms des trockenen Auges,
nach einem der Ansprüche 1 bis 10, weiter umfassend Konservierungsmittel, antimikrobielle Mittel, Sequestriermittel und Gemische davon.

12. Ophtalmische pharmazeutische Zusammensetzung zur Verwendung bei der Behandlung des Syndroms des trockenen Auges nach einem der Ansprüche 1 bis 11 in Form einer wässrigen Lösung oder Suspension in einem pharmazeutisch annehmbaren ophtalmischen Träger.

13. Ophtalmische pharmazeutische Zusammensetzung zur Verwendung bei der Behandlung des Syndroms des trockenen Auges nach Anspruch 12 in Form von Augentropfen, Gel oder Creme.

14. Ophtalmische pharmazeutische Zusammensetzung zur Verwendung bei der Behandlung des Syndroms des trockenen Auges nach einem der Ansprüche 1 bis 13, wobei das trockene Auge chronischer Art ist.

## Revendications

1. Composition pharmaceutique ophtalmique utilisée pour le traitement du syndrome de l'oeil sec, comprenant de l'hydrocortisone ou un sel toléré du point de vue pharmaceutique de celui-ci, en une quantité égale ou supérieure à 0,0005 % en poids et inférieure à 0,003 % en poids et un substitut lacrymal.

2. Composition pharmaceutique ophtalmique utilisée pour le traitement du syndrome de l'oeil sec selon la revendication 1, dans laquelle le sel de l'hydrocortisone toléré du point de vue pharmaceutique est choisi dans le groupe comprenant le phosphate sodique, l'acétate, le succinate de sodium, le butyrate et le valérate.

3. Composition pharmaceutique ophtalmique utilisée pour le traitement du syndrome de l'oeil sec selon la revendication 1 ou 2, dans laquelle l'hydrocortisone ou le sel toléré du point de vue pharmaceutique de celui-ci est présent en une quantité égale ou supérieure à 0,0008 et égale ou inférieure à 0,002, de préférence égale à 0,001 % en poids.

4. Composition pharmaceutique ophtalmique utilisée pour le traitement du syndrome de l'oeil sec selon l'une quelconque des revendications 1 à 3, dans laquelle le substitut lacrymal est choisi dans le groupe comprenant les glycosaminoglycanes, les dérivés cellulosiques, les dérivés d'acide polyacrylique, les polymères hydrosolubles tolérés du point de vue physiologique par l'oeil, et leurs mélanges.

5. Composition pharmaceutique ophtalmique utilisée pour le traitement du syndrome de l'oeil sec selon la revendication 4, dans laquelle le glycosaminoglycane est le hyaluronate de sodium.

6. Composition pharmaceutique ophtalmique utilisée pour le traitement du syndrome de l'oeil sec selon l'une quelconque des revendications 1 à 5, dans laquelle le substitut lacrymal est présent en une quantité comprise entre 0,1 et 0,5 % en poids, de préférence 0,2 % en poids.

7. Composition pharmaceutique ophtalmique utilisée pour le traitement du syndrome de l'oeil sec selon l'une quelconque des revendications 1 à 6, dans laquelle le substitut lacrymal a un poids moléculaire compris entre 500 et 1 200 kDa.

8. Composition pharmaceutique ophtalmique utilisée pour le traitement du syndrome de l'oeil sec selon l'une quelconque des revendications 1 à 7, ayant un pH compris entre 5,5 et 7,5.

9. Composition pharmaceutique ophtalmique utilisée pour le traitement du syndrome de l'oeil sec selon l'une quelconque des revendications 1 à 8, ayant une osmolarité tolérée du point de vue physiologique, de préférence comprise entre 240 et 330 mOsm / kg.

10. Composition pharmaceutique ophtalmique utilisée pour le traitement du syndrome de l'oeil sec selon l'une quelconque des revendications 1 à 9, comprenant en outre au moins un composant choisi dans le groupe comprenant des agents tampons, des régulateurs d'osmolarité et leurs mélanges.

11. Composition pharmaceutique ophtalmique utilisée pour le traitement du syndrome
de l'oeil sec selon l'une quelconque des revendications 1 à 10, comprenant en outre des agents conservateurs, des agents antimicrobiens, des agents séquestrants et leurs mélanges

12. Composition pharmaceutique ophtalmique utilisée pour le traitement du syndrome de l'oeil sec selon l'une quelconque des revendications 1 à 11, sous la forme d'une solution ou suspension aqueuse dans un support ophtalmique toléré du point de vue pharmaceutique.

13. Composition pharmaceutique ophtalmique utilisée pour le traitement du syndrome de l'oeil sec selon la revendication 12, sous forme de gouttes oculaires, de gel ou de crème.

14. Composition pharmaceutique ophtalmique utilisée pour le traitement du syndrome de l'oeil sec selon l'une quelconque des revendications 1 à 13, dans laquelle le syndrome de l'oeil sec est du type chronique.
